(19) Europäisches Patentamt
European Patent Office
Office européen des brevets

(11) Veröffentlichungsnummer : **0 219 055 B1**

# (12) EUROPÄISCHE PATENTSCHRIFT

(45) Veröffentlichungstag der Patentschrift : **07.06.89**

(51) Int. Cl.⁴ : **C 07 D207/273**, C 07 D207/267

(21) Anmeldenummer : **86113940.0**

(22) Anmeldetag : **08.10.86**

## (54) Verfahren zur Herstellung von Clausenamid.

(30) Priorität : **18.10.85 DE 3537075**

(43) Veröffentlichungstag der Anmeldung : **22.04.87 Patentblatt 87/17**

(45) Bekanntmachung des Hinweises auf die Patenterteilung : **07.06.89 Patentblatt 89/23**

(84) Benannte Vertragsstaaten : **AT BE CH DE ES FR GB IT LI LU NL SE**

(56) Entgegenhaltungen :
**AT-B- 374 457**
**AT-B- 377 982**
**PLANTA MEDICA, Band 32, 1977, Stuttgart. L. MESTER et al.: "Inhaltstoffe aus Clausena anisata (Willd.)Oliv.(Rutaceae)I. Cumarine aus der Wurzelrinde", Seiten 81-85**
**PHYTOCHEMISTRY, Band 17, 1978, Oxford, New York, Frankfurt, Sydney. DHAN RAKASH et al.: "Coumarins from Clausena indica", Seiten 1194, 1195**

(73) Patentinhaber : **BAYER AG**
**D-5090 Leverkusen 1 Bayerwerk (DE)**

**CHINESE ACADEMY OF MEDICAL SCIENCES**
**Institute of Materia Medica 1 Xian Nong Tan Street**
**Beijing (CN)**

(72) Erfinder : **Hartwig, Wolfgang, Dr.**
**Pahlkestrasse 3**
**D-5600 Wuppertal 1 (DE)**

(74) Vertreter : **Dänner, Klaus, Dr. et al**
**c/o Bayer AG Konzernverwaltung RP Patentabteilung**
**D-5090 Leverkusen 1 Bayerwerk (DE)**

EP 0 219 055 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die Erfindung betrifft ein Verfahren zur Herstellung von (±)3(S*), 4(R*), 5(R*), 7(S*)-3-Hydroxy-5-α-hydroxybenzyl-1-methyl-4-phenyl-pyrrolidin-2-on (Clausenamid).

Es ist bekannt, daß Rutaceae Clausena anicata in bestimmten Teilen Afrikas als Volksmedizin verwendet wird [J. Mester et al., Planta Medica 32, 81 (1977)]. Es ist ebenfalls bekannt, daß der rohe Extrakt von Clausena indica Oliv. cardiovaskuläre Aktivität besitzt und daß zwei aus Clausena pentaphylla (Roxb.) dünnschichtchromatographisch isolierte Cumarinderivate, Clausmarin A und B, spasmolytische Aktivität besitzen [Dhan Prakash et al., J. Chem. Soc. Chem. Commun. 1978, 281]. Außerdem gilt der wäßrige Extrakt aus Blättern von Clausena Lansium (lour) Skeels in der chinesischen Volksmedizin als wirksames Leberschutzmittel und wird gegen akute und chronische Virushepatitis eingesetzt. Aus diesem Extrakt konnte als einer der Hauptbestandteile (±)3(S*), 4(R*), 5(R*), 7(S*)-3-Hydroxy-5-α-hydroxybenzyl-1-methyl-4-phenyl-pyrrolidin-2-on (Clausenamid) der Formel (I) isoliert werden.

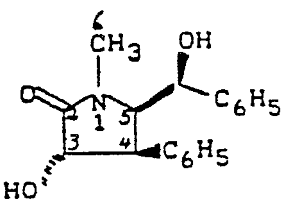

(I)

« Clausenamid »

Clausenamid zeigt im Tierversuch antiamnetische sowie vor cerebraler Hypoxie schützende Wirkung. Da für weitere pharmakologische Studien größere Mengen benötigt werden, andererseits durch das aufwendige Extraktionsverfahren aus 4 kg getrockneten Blättern nur 1,5 g Clausenamid gewonnen werden, war es notwendig ein Verfahren zur chemischen Synthese von Clausenamid zur Verfügung zu stellen.

Die vorliegende Erfindung betrifft ein Verfahren zur Herstellung von (±)3(S*), 4(R*), 5(R*), 7(S*)-3-Hydroxy-5-α-hydroxybenzyl-1-methyl-4-phenyl-pyrrolidin-2-on (I), dadurch gekennzeichnet, daß man (±)4(R*), 5(R*), 7(S*)-5-α-Hydroxybenzyl-1-methyl-4-phenylpyrrolidin-2-on der Formel (II)

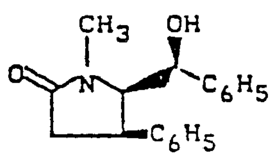

(II)

in inerten organischen Lösungsmitteln in Anwesenheit einer Base, gegebenenfalls in Gegenwart eines Hilfsstoffes oxidiert.

Es ist als ausgesprochen überraschend zu bezeichnen, daß mit Hilfe des erfindungsgemäßen Verfahrens ausschließlich das richtig konfigurierte C(3)-C(4)-trans-Hydroxylierungsprodukt (I) in guter Ausbeute entsteht. Das Produkt ist mit dem aus Pflanzenextrakt gewonnenen Clausenamid identisch. Mit dem neuen Verfahren können im Vergleich zum Extraktionsverfahren größere Mengen in kürzerer Zeit und mit weniger Aufwand zur Verfügung gestellt werden. Außerdem ist so die Verunreinigung des Clausenamids durch weitere nur schwierig abzutrennende pflanzliche Wirkstoffe ausgeschlossen.

Der Reaktionsablauf läßt sich durch folgende Gleichung wiedergeben :

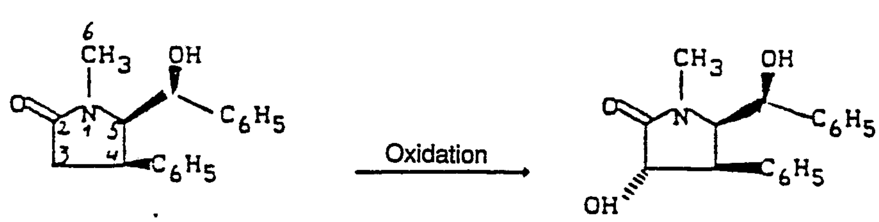


(II) (I)

Als Oxidationsmittel können organische oder anorganische Peroxoverbindungen wie beispielsweise Peroxoessigsäure, Chlorperbenzoesäure oder der Molybdänperoxid/Pyridinkomplex, außerdem Sauerstoff, Ozon oder Sauerstoffüberträger wie beispielsweise 2-Sulfonyloxaziridin eingesetzt werden.

Als Lösungsmittel kommen die üblichen inerten organischen Lösungsmittel in Frage, die sich unter den Reaktionsbedingungen nicht verändern. Hierzu gehören vorzugsweise Kohlenwasserstoffe wie beispielsweise Benzol, Toluol, Xylol, Hexan, Cyclohexan oder Erdölfraktionen, Ether wie beispielsweise Diethylether, Tetrahydrofuran oder Dioxan, Alkohole wie beispielsweise Methanol, Ethanol, Isopropyl

oder Propanol, Halogenkohlenwasserstoffe wie beispielsweise Dichlormethan, Trichlormethan, Tetrachlormethan oder 1,2-Dichlorethan oder Eisessig, Acetonitril oder Hexamethyl-phosphorsäuretriamid. Ebenso ist es möglich, Gemische der genannten Lösungsmittel zu verwenden.

Als Basen können die zur Enolatbildung üblichen Basen verwendet werden. Hierzu gehören bevorzugt Alkalialkoholate, Alkaliamide, Alkalihydride oder Alkaliorganoverbindungen wie beispielsweise Natrium- bzw. Kaliummethanolat, Natrium- bzw. Kaliumethanolat, Kalium-tert-butanolat, Natriumhydrid, Natriumamid, Lithiumdiisopropylamid, Butyllithium oder Phenyllithium. Ebenso können tertiäre Amine wie beispielsweise 1,5-Diazabicyclo(4,3,0) non-5-en oder 1,8-Diazabicyclo(5,4,0) undec-7-en eingesetzt werden. Besonders bevorzugte Basen sind Lithiumdiisopropylamid, Lithiumhexamethylpiperidid und n-, sec-, tert-Butyl- bzw. Phenyllithium.

Die Wahl der Base, des Lösungsmittels sowie gegebenenfalls des Hilfsstoffes richtet sich nach der ausgewählten Oxidationsmethode.

Als Hilfsstoffe werden gegebenenfalls Stoffe eingesetzt, die in situ entstehende Hydroperoxidzwischenstufen zu reduzieren vermögen, insbesondere bei der Verwendung von Molybdänperoxid, Pyridin oder Sauerstoff als Oxidationsmittel. Bevorzugt werden hierfür Phosphite verwendet, insbesondere Trialkyl- oder Triarylphosphite wie beispielsweise Trimethylphosphit, Triethylphosphit, Tripropylphosphit, Triisopropylphosphit, Tributylphosphit oder Triphenylphosphit.

Besonders geeignet ist die Oxidation mit Molybdänperoxid/Pyridin in Hexamethylphosphorsäuretriamid sowie mit Sauerstoff, jeweils unter Verwendung von Phosphiten. Ganz besonders gute Ausbeuten erhält man bei der Oxidation mit Sauerstoff in einem Lösungsmittel wie Tetrahydrofuran oder Hexamethylphosphorsäuretriamid, gegebenenfalls Gemische derselben, unter Verwendung von Triethylphosphit. Es hat sich hierbei als günstig erwiesen, Lithiumdiisopropylamid oder Butyllithium als Base zu verwenden.

Die Reaktionstemperaturen können zwischen — 100 °C und + 20 °C variiert werden. Bevorzugt wird zwischen — 80 °C und 0 °C gearbeitet.

Die Hydroxylierung nach dem erfindungsgemäßen Verfahren kann bei normalem aber auch bei erhöhtem oder erniedrigtem Druck durchgeführt werden. Im allgemeinen arbeitet man bei Normaldruck.

Bei der Durchführung des erfindungsgemäßen Verfahrens werden pro Mol der Ausgangsverbindung 1 bis 5, bevorzugt 1 bis 2,5 Mol Base sowie 0,5 bis 5, bevorzugt 0,5 bis 2 Mol des Hilfsstoffes eingesetzt.

Üblicherweise stellt man zunächst in dem geeignetsten Lösungsmittel mit Hilfe der Base das Enolat von II her und leitet durch die Lösung unter Zusatz von Phosphit solange absoluten Sauerstoff, bis dünnschichtchromatographisch keine Veränderung mehr zu erkennen ist. Die Aufarbeitung des Reaktionsgemisches erfolgt in üblicher, dem Fachmann bekannter Weise.

Die Ausgangsverbindung der Formel (II) ist neu. Sie kann hergestellt werden, indem man (±) (4R*), 5(R*)-5-Formyl-1-methyl-4-phenyl-pyrrolidin-2-on der Formel (III)

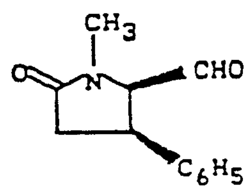

(III)

in geeigneten Lösungsmitteln mit metallorganischen Verbindungen wie Grignard-Reagenzien, Titan- oder Lithium- organylen in einem Temperaturbereich von — 20 °C bis + 50 °C, bevorzugt von — 10 °C bis + 30 °C umsetzt und das Produkt gegebenenfalls am Kohlenstoffatom 7 epimerisiert.

Als metallorganische Verbindungen eignen sich hierbei besonders Phenylmagnesiumbromid oder -chlorid oder Phenyl-triisopropoxytitan.

Als Lösungsmittel eignen sich alle inerten organischen Lösungsmittel, die bei Reaktionen mit Grignard- oder anderen metallorganischen Reagenzien üblicherweise verwendet werden. Hierzu gehören bevorzugt Ether wie Diethylether oder Tetrahydrofuran, gegebenenfalls vermischt mit Hexan.

Die Umsetzung kann in Analogie zu literaturbekannten Verfahren durchgeführt werden, wie sie beispielsweise von D. Seebach, B. Weidmann und L. Widler in « Modern Synthetic Methods 1983 » Seite 217 ff (Verlag Salle und Sauerländer) oder in Houben-Weyl's « Methoden der organischen Chemie » Band XIII/2a, S. 289 ff. S. 302 ff oder von N. L. Drake und G. B. Cooke in Organic Synthesis, Coll. Vol. II, 406 ff (1963) beschrieben sind.

Je nach Art des verwendeten metallorganischen Reagenzes kann zunächst das am Kohlenstoffatom 7R*-konfigurierte (±)4(R*), 5(R*), 7(R*)-5-α-Hydroxybenzyl-1-methyl-4-phenyl-pyrrolidin-2-on der Formel (IIa) entstehen

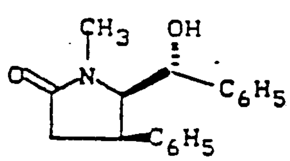

(IIa)

das durch Oxidation zu (±)4(R*), 5(R*)-5-Benzoyl-1-methyl-4-phenylpyrrolidin-2-on (IV)

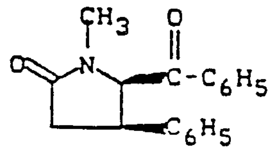

(IV)

und anschließende Reduktion von IV zum 7-R*-konfiguriertem Produkt (II) epimerisiert wird.

Die Oxidation von IIa zu IV erfolgt in Analogie zu bekannten Verfahren mit Dimethylsulfoxid als Oxidationsmittel, unter Zugabe von Anhydrieden, insbesondere von Trifluoracetanhydrid, in geeigneten organischen Lösungsmitteln, insbesondere in Halogenkohlenwasserstoffen wie z. B. Dichlormethan, Chloroform oder Kohlenwasserstoffen wie Benzol, Toluol, Xylol, Hexan oder Ethern wie Diethylether, Dioxan oder Tetrahydrofuran, oder aber in Gemischen der genannten Lösungsmitteln, wie beispielsweise von S. L. Huang, K. Omura und D. Swern in Synthesis 1980, 297 beschrieben wird.

Die Reduktion von IV zu II kann mit den üblichen Reduktionsmitteln durchgeführt werden. Besonders geeignet sind hierfür Metallhydride und komplexe Metallhydride wie beispielsweise Lithiumboranat, Lithiumhydridoborate, Natriumhydridoborate, Borane, Natriumhydridoaluminate, Lithiumhydridoaluminate oder Zinnhydride. Besonders bevorzugt werden Lithiumhydridoborate wie z. B. Lithium-hydridotriethyl-borat, Lithium-hydrido-tris(1-methylpropyl) borat oder Natriumborhydrid eingesetzt.

Als Lösungsmittel eignen sich die üblichen, bei Reduktionen mit Hydriden verwendeten inerten organischen Lösungsmittel. Bevorzugt sind dies Ether wie Diethylether und Tetrahydrofuran. Die Reduktion wird in Analogie zu bekannten Methoden durchgeführt [W. Friedrichsen in Houben-Weyl, « Methoden der organischen Chemie » VIII/1b, 145 ff ; H. C. Brown, S. Krishnamurthy, Chem. Commun. 1972, 868].

Außerdem kann die Verbindung (IIa) auch in Analogie zu anderen bekannten Verfahren epimerisiert werden, wie z. B. von O. Mitsunobu in Synthesis 1981, 1 beschrieben.

Die Herstellung von II kann durch folgendes Formelschema verdeutlicht werden :

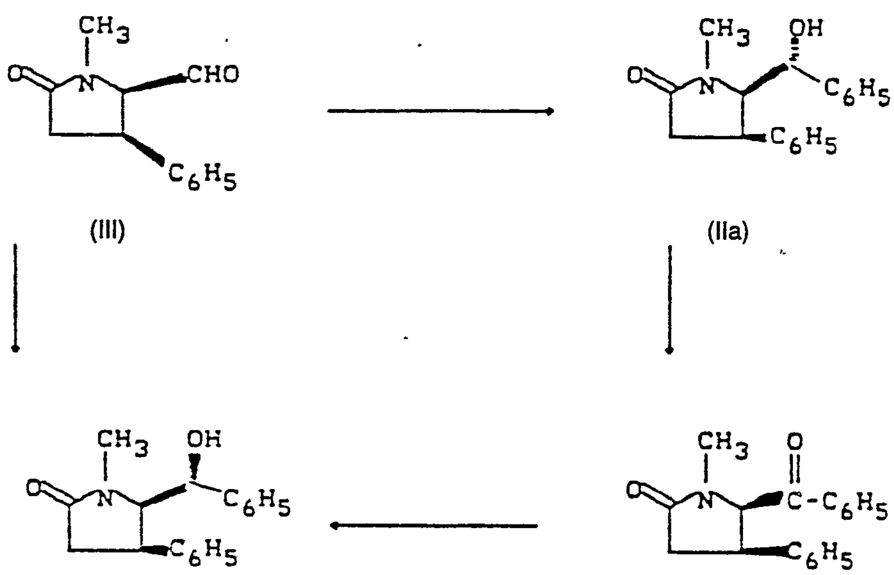

(III) (IIa) (II) (IV)

Verwendet man als metallorganisches Reagenz das leicht zugängliche Phenylmagnesiumbromid, entsteht fast ausschließlich das « falsch » konfigurierte IIa, das in angegebener Weise zum « richtig » konfigurierten II epimerisiert wird.

Die vorliegende Erfindung betrifft ebenfalls die neuen Verbindungen der Formeln IIa, IV sowie III.

Der Aldehyd der Formel III kann gemäß folgendem Formelschema hergestellt werden :

(Siehe Formelschema Seite 5 f.)

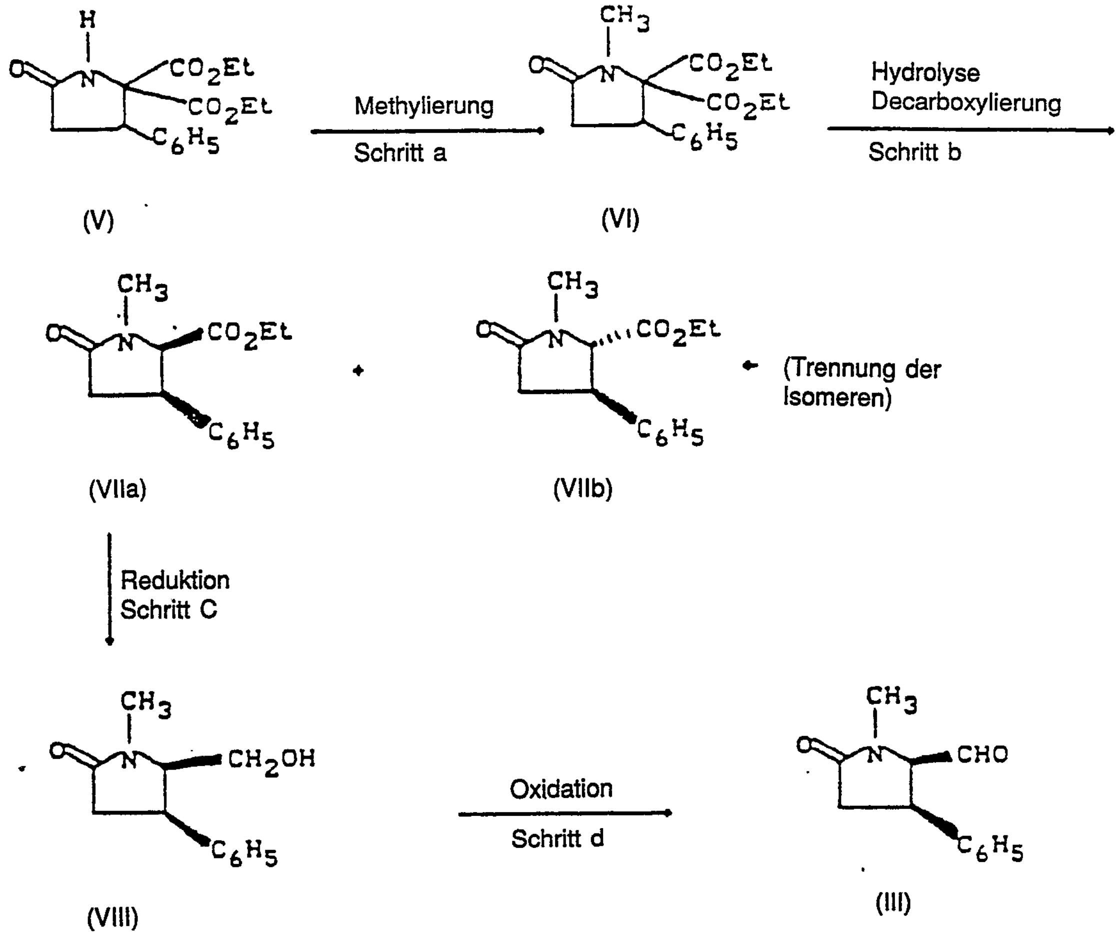


(V) (VI) (VIIa) (VIIb) (VIII) (III)

Danach wird in Schritt a) 5,5-Diethoxycarbonyl-4-phenyl-pyrrolidin-2-on (V) mit einem Methylierungsmittel wie beispielsweise Methylbromid, Methyliodid, Methyl-p-toluol-sulfonat, Diazomethan oder Dimethylsulfat, gegebenenfalls in Anwesenheit einer Base wie Natrium, Natriumhydrid, Natriumamid, Butyllithium oder Lithiumdiisopropylamid in geeigneten Lösungsmitteln wie Diethylether, Tetrahydrofuran, Dimethylformamid oder Hexamethylphosphorsäuretriamid bei Temperaturen von —20 bis +80 °C, bevorzugt von 0 °C bis +40 °C methyliert. Ganz besonders geeignet ist die Methylierung mit Methyliodid in Dimethylformamid. Es hat sich hierbei als günstig erwiesen, Natriumhydrid als Base zu verwenden. Durchführung und Aufarbeitung erfolgt nach üblichen, dem Fachmann geläufigen Methoden.

In Schritt b) wird 5,5-Diethoxycarbonyl-1-methyl-4-phenyl-pyrrolidin-2-on (VI) in Analogie zu dem von P. Pachaly in Chem. Ber. 104, (2), 412-39 (1971) beschriebenen Verfahren hydrolysiert und decarboxyliert, wobei ein Gemisch der Isomeren VIIa und VIIb anfällt. Nach Trennung der cis/-trans-Isomeren VIIa und b durch Umkristallisieren oder Chromatographieren wird VIIa zu (±)4(R*), 5(R*)-5-Hydroxymethyl-1-methyl-4-phenylpyrrolidin-2-on (VIII) reduziert (Schritt c).

Die Reduktion von VIIa zu VIII (Schritt c) erfolgt nach der gleichen Methoden und unter den gleichen Bedingungen wie bereits für die Reduktion von IV zu II angegeben.

Die Oxidation von VIII zu III (Schritt d) erfolgt nach der gleichen Methode und unter den gleichen Bedingungen, wie bereits für die Oxidation von IIa zu IV angegeben.

Die Ausgangsverbindung V ist literaturbekannt [G. H. Cocolas, W. H. Hartung, J. Am. Chem. Soc. 79, 5203 (1957) ; F. Zymalkowski, P. Pachaly, Chem. Ber. 100, 1137 (1967)].

Im folgenden Schema wird die gesamte Synthese von Clausenamid mit allen neuen Zwischenprodukten verdeutlicht :

(Siehe Formelschema Seite 6 f.)

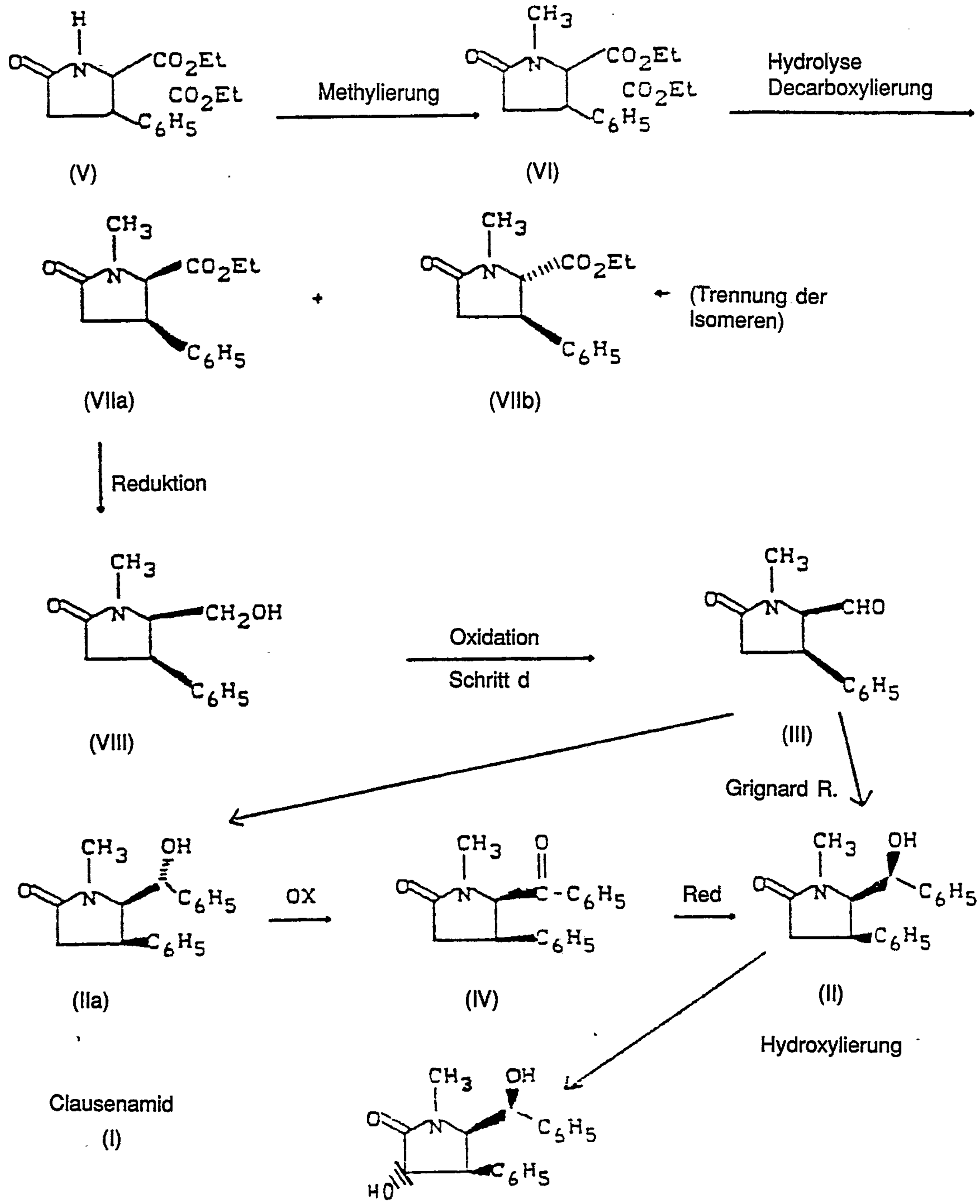


(V) (VI) (VIIa) (VIIb) (VIII) (III) (IIa) (IV) (II)

Clausenamid
(I)

Herstellungsbeispiele

Beispiel 1

(±) 5,5-Diethoxycarbonyl-4-phenylpyrrolidin-2-on

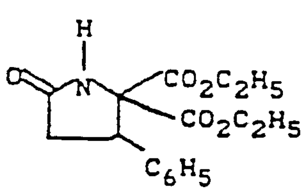

Zur Suspension von 432 g (2 Mol) Acetamidomalonsäurediethylester in 1,6 l absolutem Ethanol tropfte man bei Raumtemperatur unter $N_2$-Atmosphäre eine Lösung von 18 g (0,8 Grammatom) Natrium in 400 ml absolutem Ethanol. Man gab 564 g (3,2 Mol) Zimtsäureethylester langsam zu und erhitzte anschließend 24 h zum Sieden.

Zur Aufarbeitung ließ man auf Raumtemperatur kommen, gab 2,5 l Chloroform zu und neutralisierte mit Essigsäure. Die Mischung wurde gut mit Wasser gewaschen (5 × je 500 ml), über $MgSO_4$ getrocknet und einrotiert. Der ölige Rückstand wurde in wenig Aceton gelöst, mit Hexan bis zur Kristallisation versetzt und anschließend weiter Hexan zugegeben bis an der Eintropfstelle keine Trübung mehr zu beobachten war. Absaugen lieferte 398 g (54 %) der Titelverbindung vom Schmp. 97-99 °C. Chromatographie der Mutterlauge (Toluol/Essigester) ergab weitere 85 g (14 %) der Titelverbindung Gesamtausbeute 413 g (68 %).

IR (KBr) : V = 1 770 (Ester), 1 700 (Amid).

$^1$H-NMR (300 MHz, $CDCl_3$) ; δ = 0,84 und 1,28 (je t, J = 7,5 Hz ; 6H, $CH_2CH_3$) ; ABX Signal ; $\delta_A$ = 2,63, $\delta_B$ = 2,96 ($J_{AB}$ = 17,3 Hz, $J_{AX}$ = 6 Hz, $J_{BX}$ = 9 Hz ; 2H, C(3)-H) ; 3,66 und 3,71 (je m, 2H, cis-$CH_2$-$CH_3$) ; 4,28 (m, 2H, trans-$CH_2$-$CH_3$) ; 4,39 (dd, $J_{AX}$ = 6 Hz, $J_{BX}$ = 9 Hz, 1H, C(4)-H) ; 6,95 (br, 1H, NH) ; 7,39 (br, 5H, $C_6H_5$).

Beispiel 2

(±) 5,5-Diethoxycarbonyl-1-methyl-4-phenyl-pyrrolidin-2-on

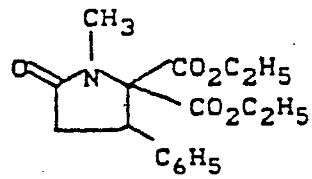

Zur Suspension von 9,64 g (0,36 Mol) Natriumhydrid in 200 ml absolutem Dimethylformamid tropfte man bei Raumtemperatur unter $N_2$-Atmosphäre die Lösung von 100 g (0,33 Mol) (±) 5,5-Diethoxycarbonyl-4-phenylpyrrolidin-2-on in 500 ml absolutem Dimethylformamid. Man ließ so lange bei Raumtemperatur nachrühren, bis die Gasentwicklung beendet war, gab anschließend die Lösung von 93,7 g (0,66 Mol) Methyliodid in 50 ml absolutem Dimethylformamid zu und rührte bei Raumtemperatur bis alles Ausgangsmaterial umgesetzt war (ca. 1 h, DC Kontrolle). Man goß die Reaktionsmischung in 2 l Pufferlösung pH = 7 und extrahierte 5 mal mit je 600 ml Diethylether. Trocknen der organischen Extrakte ($MgSO_4$) und Abziehen des Solvens im Vakuum ergaben 105 g (99,6 %) der Titelverbindung (nach $^1$H-NMR Spektrum zu 95 % rein) die direkt weiter umgesetzt wurden. Zur Analyse wurde eine Probe am Kugelrohr destilliert ($K_{P0,5}$ : 240 °C) $R_f$ : 0,36 (Toluol/Essigester : 2/1).

IR (Film) ; v = 1 735 (Ester) ; 1 700 (Amid).

$^1$H-NMR (500 MHz, $CDCl_3$) ; δ = 0,9 und 1,33 (je t, J = 7,5 Hz ; 6H, $CH_2CH_3$) ; ABX-Signal : $\delta_A$ = 2-66 ; $\delta_B$ = 3-0 ($J_{AB}$ = 18 Hz, $J_{AX}$ = 6 Hz, $J_{BX}$ = 8-3 Hz ; 2H, C(3)-H) ; 3-06 (s ; 3H, N-$CH_3$) ; 3,62 und 3,79 (je m, 2H, cis-$CH_2$-$CH_3$) ; 4,31 (m, 3H, trans-$CH_2$-$CH_3$) und C(4)-H) ; 7,26 (m, 5H, $C_6H_5$)

Beispiel 3

(±)4(R*), 5(R*)-[I] und (±)4(R*), 5(S*)-5-Ethoxycarbonyl-1-methyl-4-phenylpyrrolidin-2-on [II]

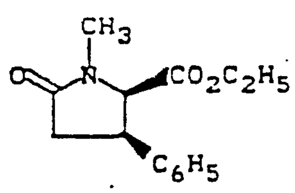

(I)

+

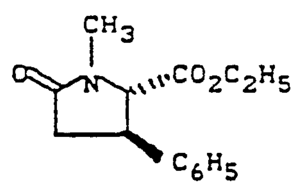

(II)

49,5 g (0,156 Mol) Bariumhydroxid-octahydrat werden in 483 ml destilliertem Wasser auf 70 °C bis zur nahezu klaren Lösung erwärmt. Man gab die Lösung von 100 g (0,313 Mol) (±)5,5-Diethoxycarbonyl-1-methyl-4-phenylpyrrolidin-2-on in 724 ml Ethanol zu (klare Lösung) und rührte 20 min. bei 70 °C nach, bis das Ausgangsmaterial vollständig umgesetzt war (ca. 20 Minuten, DC-Kontrolle). Man kühlte ab, säuerte unter Eiskühlung auf pH = 1-2 an und zog das Ethanol im Vakuum ab (Badtemperatur 30-40 °C). Man saugte den Festkörper ab und extrahierte die wäßrige Phase unter Kochsalz-Zusatz 3 mal mit je 200 ml Essigester. Trocknen und Abziehen des Solvens lieferte einen Rückstand, der mit dem oben erhaltenen Festkörper vereinigt und 24 h in Exsikkator über $P_4O_{10}$ im Hochvakuum getrocknet wurde. Anschließend

wurde der Festkörper unter gutem Rühren im Ölbad auf 170 °C erwärmt, bis die Gasentwicklung beendet war (5-10 Min). Abkühlen und flash-Chromatographie (Cyclohexan/Essigester = 1/1, zum Schluß mit Essigester) lieferten 39,3 g (50,7 %) (I) mit $R_f$ = 0,10 und 19,6 g (25,3 %) II mit $R_f$ = 0,20 (jeweils in Cyclohexan/Essigester 1/1).

IR(KBr) : δ = 1 736, 1 690 $cm^{-1}$.

$^1$H-NMR (200 MHz, $CDCl_3$) ; I : δ = 0,83 (t, J = 7,5 Hz ; 3H, $CH_2CH_3$) ; ABX Signal : $δ_A$ = 2,67, $δ_B$ = 2,95 ($J_{AB}$ = 17,5 Hz, $J_{AX}$ = 9 Hz, $J_{BX}$ = 10 Hz ; 2H, C(3)-H) ; 2,87 (s, 3H, N-$CH_3$), 3,75 (m, 2H, $CH_2CH_3$) ; 3,91 (q, J = 9-10 Hz, 1H, C(4)-H), 4,36 (d, J = 9 Hz, 1H, C(5)-H), 7,28 (m, 5H, $C_6H_5$). II : δ = 1,30 (t, J = 7,5 Hz, 3H, $CH_2CH_3$) ; ABX-Signal : $δ_A$ = 2,54, $δ_B$ = 2,82 ($J_{AB}$ = 18,5 Hz, $J_{AX}$ = 5 Hz, $J_{BX}$ = 9 Hz, 2H, C(3)-H), 3,80 (s, 3H, N-$CH_3$), 3,53 (ddd, J = 9 Hz, J = 5 Hz, J = 4 Hz, 1H, C(4)-H), 4,07 (d, J = 4 Hz, 1H, C(5)-H), 4,27 (m, 2H, $CH_2CH_3$), 7,3 (m, 5H, $C_6H_5$).

## Beispiel 4

(±)4(R*), 5(R*)-5-Hydroxymethyl-1-methyl-4-phenylpyrrolidin-2-on

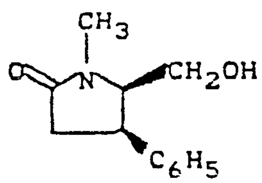

Zur Lösung von 39,2 g (0,159 Mol) cis-4(R*), 5(R*)-5-Ethoxycarbonyl-1-methyl-4-phenylpyrrolidin-2-on in 390 ml absolutem Tetrahydrofuran tropfte man bei —15 bis —20 °C unter $N_2$-Atmosphäre 0.137 Mol $LiB(Et)_3H$ (als 1 M Lösung in Tetrahydrofuran, 316,9 ml).

Man ließ 1 h bei 0 °C nachrühren, goß die Reaktionsmischung in ca. 200 ml eiskalte 2 N Salzsäure und extrahierte zweimal mit je 200 ml Essigester. Man sättigte die wäßrige Phase mit Kochsalz und extrahierte nochmals zweimal mit je 200 ml Essigester. Die gesammelten organischen Extrakte wurden mit wenig Wasser gewaschen, über $MgSO_4$ getrocknet und einrotiert. Der Rückstand wurde mit wenig Ether zur Kristallisation gebracht und anschließend mit Pentan ausgefällt, bis an der Eintropfstelle keine Trübung mehr zu beobachten war. Nach Absaugen und Trocknen erhielt man 29,1 g (89,2 %) der Titelverbindung mit Schmelzpunkt 93-95 °C.

IR(KBr) : $\nu$ = 3 324, 1 687 $cm^{-1}$

$^1$H-NMR (300 Mhz, $CDCl_3$) : δ = AB-Teil vom ABM-System, $δ_A$ = 2,59, $δ_B$ = 2,97 (je dd, $J_{AB}$ = 15 Hz, $J_{AM}$ = 7,5 Hz, $J_{BM}$ = 9 Hz, 2H, C(3)-H) ; 2,97 (s, 3H, N-$CH_3$) ; AB-Teil vom ABM-System, $δ_A$ = 3,36, $δ_B$ = 3,62 (je dd, $J_{AB}$ = 11,2 Hz $J_{AM}$ = $J_{BM}$ = 3 Hz, 2H, C(7)-H) ; 3,72-3,85 (m, 2H, C(4)-H, C(5)-H) ; 7,32 (m, 5H, $C_6H_5$).

## Beispiel 5

4(R*), 5(R*)-5-Formyl-1-methyl-4-phenylpyrrolidin-2-on

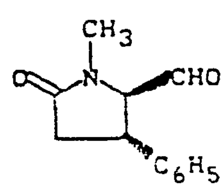

Zur Lösung von 19,9 ml (0,29 Mol) absolutem Dimethylsulfoxid in 140 ml absolutem Dichlormethan tropfte man unter $N_2$-Atmosphäre innerhalb 10 min bei —60 °C die Lösung von 29,7 ml Trifluoracetanhydrid in 56 ml absolutem Dichlormethan. Man ließ 15 min bei dieser Temperatur rühren und tropfte die Lösung von 28,8 g (0,140 Mol) 4(R*), 5(R*)-5-Hydroxymethyl-1-methyl-4-phenylpyrrolidin-2-on in 250 ml Dichlormethan so zu, daß die Temperatur —60 °C nicht überstieg. Man ließ 90 min. bei —60 °C nachrühren, erwärmte kurz auf —30 °C (5-10 min) und kühlte wieder auf —60 °C ab. Man gab langsam bei dieser Temperatur 56 ml absolutes Triethylamin zu, ließ 30 min bei —60 °C rühren und erwärmte auf Raumtemperatur. Man gab 600 ml Wasser zu, trennte die Phasen und extrahierte die wäßrige Phase dreimal mit je 250 ml Dichlormethan. Die gesammelten organischen Extrakte wurden zweimal mit je 300 ml Wasser gewaschen, über Magnesiumsulfat getrocknet und extrahiert. Man erhielt 28,3 g der Titelverbindung mit $R_f$ = 0,25 (Essigester) (nach $^1$H-NMR-Spektrum zu 91 % rein). Das so erhaltene Rohprodukt wurde nach Trocknung (24 h, HV) direkt weiter umgesetzt.

IR($CHCl_3$) : $\nu$ = 1 734, 1 689 $cm^{-1}$

$^1$H-NMR (300 MHz, $CDCl_3$) : δ 2,79 (dd, J = 5,3 Hz, J = 9,7 Hz, 2H, C(3)-H) ; 2,91 (S, 3H, N-$CH_3$) ; 4,02

(q, J = 9,7 Hz, 1H, C(4)-H) ; 4,30 (dd, J = 1 Hz, J = 9,7 Hz, 1H, C(5)-H) ; 7,3 (m, 5H, $C_6H_5$), 9,17 (d, J = 1 Hz, 1H, CHO).

Beispiel 6

4(*), 5(R*), 7(*)-5-α-Hydroxybenzyl-1-methyl-4-phenylpyrrolidin-2-on

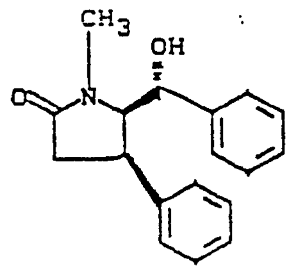

Zu 3,84 g Mg-Späne tropfte man unter $N_2$-Atmosphäre die Lösung von 24,8 g (16,7 ml), 0,156 Mol) Brombenzol in 44 ml absolutem Tetrahydrofuran so zu, daß das Tetrahydrofuran gelinde siedete. Man gab anschließend 100 ml absolutes Tetrahydrofuran zu und erhitzte unter Rückfluß zum Sieden bis alles Magnesium gelöst war (1-2 h).

Man kühlte auf 0 °C und tropfte unter kräftigem Rühren die Lösung von 24,7 g (0,12 Mol) 4(S*), 5(R*)-5-Formyl-1-methyl-4-phenylpyrrolidin-2-on in 250 ml absolutem Tetrahydrofuran so zu, daß die Temperatur 5 °C nicht überstieg. Gegebenenfalls mußte absolutes Tetrahydrofuran zur besseren Rührfähigkeit zugegeben werden. Man rührte anschließend 1 h bei 0-5 °C und goß die Reaktionsmischung auf 350 ml 0,5 NHCl-Eis und extrahierte viermal mit je 300 ml Essigester und zweimal mit je 300 ml Dichlormethan. Die gesammelten Essigester- und Dichlormethan-Extrakte wurden (getrennt !) zweimal mit je 200 ml Wasser gewaschen, vereinigt und über Magnesiumsulfat getrocknet. Der nach Abziehen des Solvens (im Vakuum) verbleibende Rückstand wurde mit 100 ml Ether bis zur Kristallisation verrieben. Anschließend gab man 500 ml Pentan langsam zu und ließ über Nacht im Kühlschrank stehen. Absaugen des Festkörpers ergab 25 g (74,3 %) der Titelverbindung mit Schmelzpunkt : 210-212 °C.

Zur Analyse wurde aus Aceton umkristallisiert (Schmelzpunkt 214-5 °C).

IR(KBr) $\nu$ = 3 362 (br), 1 654 $cm^{-1}$

$^1$H-NMR (300 MHz, $d_6$-DMSO) : $\delta$ = 2,21 (s, 3H, $NCH_3$) ; 2,24 (dd, A-Teil von ABM-System, $J_{AB}$ = 15,7 Hz, $J_{AM}$ = 9,4 Hz, 1H, cis-C(3)-H) ; 3,05 (dd, B-Teil von ABM-System, $J_{BM}$ = 12,7 Hz, 1H, trans-C(3)-H) ; 3,80 (dt, M-Teil von ABM-System, $J_{AM}$ = 8,5 Hz, $J_{AB}$ = 12,7 Hz, $J_{4,5}$ = 8,5 Hz, 1H, C(4)-H) ; 4,15 (dd; J = 8,5 Hz, J = 1 Hz, 1H, C(5)-H) ; 4,26 (dd, J = 6 Hz, J = 1 Hz, 1H, C(7)-H) ; 5,35 (d, J = 6 Hz, 1H, OH) ; 7,15-7,5 (m, 10H, $C_6H_5$).

Beispiel 7

4(R*), 5(R*)-5-Benzoyl-1-methyl-4-phenylpyrrolidin-2-on

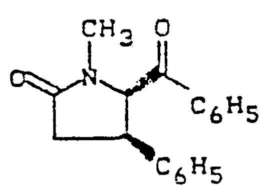

Zur Lösung von 12,24 ml (0,171 Mol) absolutem Dimethylsulfoxid in 87 ml absolutem Dichlormethan tropfte man unter $N_2$-Atmosphäre innerhalb 10 min bei — 60 °C die Lösung von 18 ml Trifluoracetanhydrid in 34 ml absolutem Dichlormethan. Man ließ 15 min bei dieser Temperatur nachrühren und tropfte die Lösung von 24 g (0,085 Mol) 4(R*), 5(R*), 7(R*)-5-α-Hydroxy-benzyl-1-methyl-4-phenylpyrrolidin-2-on in ca. 700 ml absolutem Dichlormethan so zu, daß die Temperatur — 60 °C nicht überstieg. Man ließ 90 min bei — 60 °C nachrühren, erwärmte kurz auf — 30 °C (9-10 min) und kühlte wieder auf — 60 °C ab.

Man gab langsam bei dieser Temperatur 34,2 ml Triethylamin zu, ließ 20 min bei — 60 °C rühren und erwärmte auf Raumtemperatur. Man gab 370 ml Wasser zu, trennte die Phasen und extrahierte die wäßrige Phase dreimal mit je 250 ml Dichlormethan. Die vereinigten organischen Extrakte wurden zweimal mit je 300 ml Wasser gewaschen, über Magnesiumsulfat getrocknet und einrotiert. Der Rückstand wurde zweimal mit je 200 ml Ether abrotiert. Man erhielt 23,5 g (100 %) der Titelverbindung als Festkörper mit Schmelzpunkt ; 115-116 °C. Das Rohprodukt, das nach $^1$H-NMR Spektrum rein war, wurde direkt weiter umgesetzt.

Zur Analyse wurde eine Probe über Kieselgel mit Essigester chromatographiert ($R_f$ = 0,25)
Schmelzpunkt : 121-2 °C
IR(KBr) : ν = 1 695, 1 682 $cm^{-1}$
$^1H$-NMR (300 MHz, $COCl_3$) : δ = 2,78 und 2,91 (AB-Teil vom ABM-Spektrum, $J_{AB}$ = 16,5 Hz, $J_{AM}$ = $J_{BM}$ = 8,3 Hz, 2H, C(3)-H) ; 2,88 (s, 3H, N-$CH_3$) ; 4,02 (q, J = 8,3 Hz, 1H, C(4)-H) ; 5,42 (d, J = 8,3 Hz, 1H, C(5)-H) ; 7,0, 7,21, 7,59, 7,50 (je m, 1OH, $C_6H_5$).

Beispiel 8

4(R*), 5(R*), 7(S*)-5-Hydroxymethylphenyl-1-methyl-4-phenylpyrrolidin-2-on

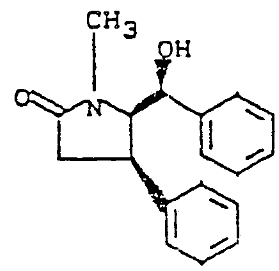

Zur Lösung von 23 g (82,3 mmol) 4(S*), 5(R*)-5-Benzoyl-1-methyl-4-phenylpyrrolidin-2-on in 200 bis 270 ml absolutem Tetrahydrofuran tropfte man bei — 15° bis — 20 °C unter $N_2$-Atmosphäre 83 mmol $LiB(Et)_3H$ (83 ml einer 1M-Lösung in Tetrahydrofuran. Man ließ 1 h bei 0 °C nachrühren, goß die Reaktionsmischung in 100 ml eiskalte 1N HCl und extrahierte zweimal mit je 200 ml Essigester. Man sättigte die wäßrige Phase mit Kochsalz und extrahierte nochmals zweimal mit je 200 ml Essigester. Die vereinigten organischen Extrakte wurden über $MgSO_4$ getrocknet und einrotiert. Den Rückstand löste man in Dichlormethan und wusch zweimal mit je 100 ml Wasser. Die organische Phase wurde getrocknet ($MgSO_4$) und einrotiert. Der Rückstand wurde mit 100 ml Ether zur Kristallisation gebracht und anschließend langsam unter Rühren mit Pentan versetzt bis an der Eintropfstelle keine Trübung mehr zu beobachten war. Der Niederschlag wurde abgesaugt und getrocknet. Man erhielt 16,6 g (72 %) der Titelverbindung mit Schmelzpunkt : 189-195 °C.

Das Produkt ist nach $^1H$-NMR zu 95 % rein und wurde direkt weiter umgesetzt.

Zur Analyse wurde aus Aceton umkristallisiert (Schmelzpunkt : 197-8 °C).
IR(KBr) : ν = 3 251, 1 692 $cm^{-1}$
$^1H$-NMR (300 MHz, DMSO) : δ = 1,97 und 2,05 (ABM-Signal, $J_{AB}$ = 13,5 Hz, $J_{AM}$ = 8,2 Hz, $J_{BM}$ = 13 Hz, 2H C(3)-H) ; 2,91 (s, 3H, N-$CH_3$) ; 3,82 (dt, $J_{AM}$ = $J_{4,5}$ = 8,2 Hz, $J_{BM}$ = 13 Hz, 1H, C(4)-H) ; 4,27 (dd, J = 8,2 Hz, J = 1,5 Hz, 1H, C(5)-H) ; 4,65 (dd, J = 1,5 Hz, J = 3,5 Hz, 1H, C(7)H) ; 5,34 (d, J = 3,5 Hz, 1H, OH) ; 6,70, 7,11, 7,25 (je m, 1OH, $C_6H_5$).

Beispiel 9

3(S*), 4(R*), 5(R*), 7(S*)-3-Hydroxy-5-α-hydroxybenzyl-1-methyl-4-phenylpyrrolidin-2-on (Clausenamid)

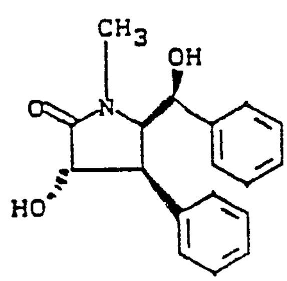

In einem im Vakuum ausgeheizten und mit Reinststickstoff belüfteten Kolben gab man die Lösung von 17,7 g (62,8 mMol) 4(R*), 5(R*), 7(S*)-5-α-Hydroxybenzyl-1-methyl-4-phenyl-pyrrolidin-2-on in 490 ml absolutem Tetrahydrofuran und 130 ml absolutem Hexamethylphosphorsäuretriamid und kühlte auf — 70 °C. Bei dieser Temperatur tropfte man die Lösung von 0,152 Mol Lithiumdiisophenylamid in 180 ml absolutem Tetrahydrofuran/Hexan zu (hergestellt aus 22,1 ml Diisopropylamin in 80 ml THF durch Zugabe von 103 ml einer 1,5 N-Lösung von n-Butyllithium in Hexan bei — 20 °C bis 0 °C). Man rührte 1 h bei — 70 °C bis — 60 °C nach, gab 5,3 ml frisch destilliertes Trimethylphosphit (gelöst in wenig absolutem Tetrahydrofuran) zu und leitete (über $H_2SO_4$ und $P_4O_{10}$ getrockneten) absoluten Sauerstoff ein (50-100 ml/min). Sobald nach DC-Kontrolle ($SiO_2$ ; EE/MeOH : 2/1 ; $R_f$ = 0,3 für Titelverbindung, $R_f$ =

0,37 für Ausgangsprodukt, Anfärben mit Molybdatophosphorsäure-Sprühreagenz) sich das Verhältnis Produkt/Ausgangsmaterial nicht mehr änderte (2-3 h) goß man unter Eiskühlung auf 600 ml 0,5 NHCl und säuerte gegebenenfalls auf pH 3 bis 4 an.

Man trennte die Phasen und extrahierte die wäßrige Phase viermal mit je 300 ml Essigester. Die vereinigten organischen Extrakte wurden dreimal mit je 300 ml Wasser gewaschen und über $MgSO_4$ getrocknet und einrotiert. Man nahm den Rückstand in 50-100 ml Ether auf, rührte bis zur beginnenden Kristallisation und gab langsam unter Rühren soviel Pentan zu, bis an der Eintropfstelle keine Trübung mehr zu beobachten war. Man ließ über Nacht im Kühlschrank stehen und saugte ab. Man erhielt ca. 17 g eines rohen Festkörpers der neben der Titelverbindung ca. 35-40 % Ausgangsmaterial enthielt. Zur Reinigung wurde zweimal aus Methanol umkristallisiert. Man erhielt dann die Titelverbindung in ca. 95 % Reinheit. Verlustfreier und mit Rückgewinnung des reinen Ausgangsmaterial verlief die Chromatographie über Aluminiumoxid (neutral). Hierzu wurde das Rohprodukt auf Kieselgel aufgezogen (Lösen in MeOH in der Wärme, Zugabe von 5 Gew.-Teilen Kieselgel, Einrotieren und mehrfaches Abrotieren mit Essigester, bis ein staubtrockenes MeOH-freies Produkt resultiert). Das Adsorbat wurde auf eine Säule mit $Al_2O_3$ (neutral, 50 Gew.-Teile) gegeben und mit Essigester zunächst das Ausgangsmaterial eluiert (flash-Chromatographie, Kontrolle mit DC und analyt. HPLC). Anschließend eluierte man die Titelverbindung mit Essigester/Methanol-Gemischen (40/1, 20/1, dann 10/1). Man erhielt 8,6 g (46,1 %) der Titelverbindung mit Schmelzpunkt: 236-7,5 °C (auth. Clausenamid: 236-7 °C) Reiheit ca. 98 % (nach $^1$H-NMR, enthält ca. 2 % Ausgangsmaterial). 5 g des reinen Ausgangsmaterials konnten zurückgewonnen werden.

IR(KBr): $\nu$ = 3 402, 3 321, 1 689 $cm^{-1}$

$^1$H-NMR (300 MHz, DMSO): $\delta$ = 3,01 (s, 3H, $N-CH_3$); 3,50 (dd, J = 8 Hz, J = 10,5 Hz, 1H, C(4)-H); 3,82 (dd, J = 10 Hz, J = 7 Hz, 1H, C(3)-H); 4,30 (dd, J = 8 Hz, J = 2 Hz, 1H, C(5)-H); 4,65 (dd, J = 2 Hz, J = 3 Hz, 1H, C(7)-H), 5,39 (d, J = 7 Hz, 1H, C(3)-OH); 5,45 (d, J = 3 Hz, 1H, C(7)-OH); 6,61-6,64 (m, 2H, arom. H); 7,03-7,28 (m, 8H, arom. H).

## Patentansprüche

1. Verfahren zur Herstellung von (±)3(S*), 4(R*), 5(R*), 7(S*)-3-Hydroxy-5-α-hydroxybenzyl-1-methyl-4-phenylpyrrolidin-2-on (I), dadurch gekennzeichnet, daß man (±)4(R*), 5(R*), 7(S*)-5-α-Hydroxybenzyl-1-methyl-4-phenylpyrrolidin-2-on der Formel (II)

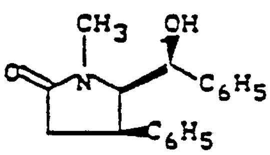

(II)

in inerten organischen Lösungsmitteln in Anwesenheit einer zur Enolatbildung geeigneten Base, gegebenenfalls in Gegenwart eines zur Reduktion von Hydroperoxiden geeigneten Hilfsstoffes mit anorganischen oder organischen Peroxoverbindungen, Sauerstoff, Ozon oder Sauerstoffüberträgern bei Temperaturen zwischen — 100 und + 20 °C oxidiert.

2. Verfahren nach Anspruch 1, dadurch gekennzeichnet, daß als zur Enolatbildung geeignete Basen tertiäre Amine verwendet werden.

3. Verfahren nach Anspruch 1, dadurch gekennzeichnet, daß man als Hilfsstoffe Phosphite verwendet.

4. Verfahren nach Anspruch 1, dadurch gekennzeichnet, daß man die Oxydation mit Sauerstoff in Tetrahydrofuran oder Hexamethylphosphorsäuretriamid oder Gemischen dieser Lösungsmittel in Gegenwart von Triethylphosphit durchführt.

5. (±)4(R*), 5(R*), 7(S*)-5-α-Hydroxybenzyl-1-methyl-4-phenylpyrrolidin-2-on der Formel (II)

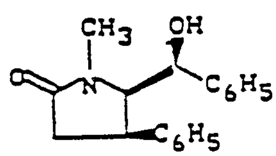

(II)

## Claims

1. Process for the preparation of (±)3(S*), 4(R*), 5(R*), 7(S*)-3-hydroxy-5-α-hydroxybenzyl-1-methyl-4-phenylpyrrolidin-2-one (I), characterised in that (±)4(R*), 5(R*), 7(S*)-5-α-hydroxybenzyl-1-methyl-4-phenylpyrrolidin-2-one of the formula (II)

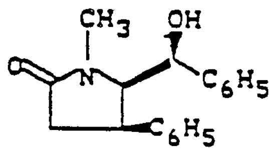

(II)

is oxidised in inert organic solvents in the presence of a base suitable for enolate formation, if appropriate in the presence of an auxiliary suitable for the reduction of hydroperoxides, using inorganic or organic peroxo compounds, oxygen, ozone or oxygen transfer agents at temperatures between —100 and + 20 °C.

2. Process according to Claim 1, characterised in that tertiary amines are used as bases suitable for enolate formation.

3. Process according to Claim 1, characterised in that phosphites are used as auxiliaries.

4. Process according to Claim 1, characterised in that the oxidation is carried out with oxygen in tetrahydrofuran or hexamethylphosphoric acid triamide or mixtures of these solvents, in the presence of triethyl phosphite.

5. (±)4(R*), 5(R*), 7(S*)-5-α-Hydroxybenzyl-1-methyl-4-phenylpyrrolidin-2-one of the formula (II)

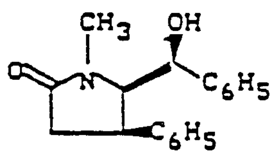

(II)

**Revendications**

1. Procédé de préparation de la (±)3(S*), 4(R*), 5(R*), 7(S*)-3-hydroxy-5-α-hydroxybenzyl-1-méthyl-4-phénylpyrrolidine-2-one (I), caractérisé en ce que l'on oxyde à des températures allant de —100 à + 20 °C la (±)4(R*), 5(R*), 7(S*)-5-α-hydroxybenzyl-1-méthyl-4-phénylpyrrolidine-2-one de formule II

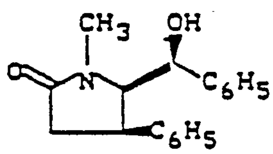

(II)

dans des solvants organiques inertes en présence d'une base appropriée à la formation d'un énolate, éventuellement en présence d'un produit auxiliaire convenant pour la réduction des hydropéroxydes, à l'aide de composés minéraux ou organiques en peroxo, d'oxygène, d'ozone ou d'agents de transfert de l'oxygène.

2. Procédé selon la revendication 1, caractérisé en ce que l'on utilise en tant que bases appropriées à la formation d'énolates des amines tertiaires.

3. Procédé selon la revendication 1, caractérisé en ce que l'on utilise en tant que produits auxiliaires des phosphites.

4. Procédé selon la revendication 1, caractérisé en ce que l'oxydation à l'aide de l'oxygène est effectuée dans le tétrahydrofuranne ou l'hexaméthylphosphorotriamide ou dans des mélanges de ces solvants en présence de phosphite de triéthyle.

5. La (±)4(R*), 5(R*), 7(S*)-5-α-hydroxybenzyl-1-méthyl-4-phénylpyrrolidine-2-one de formule II

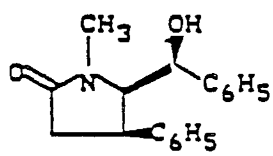

(II)